# EUROPEAN PATENT SPECIFICATION

(11) **EP 2 661 280 B1**
(45) Date of publication and mention of the grant of the patent: **21.11.2018**
(21) Application number: 12700455.4
(22) Date of filing: 03.01.2012
(51) Int. Cl.: A61K 47/24, A61K 9/107

(54) **O/W-EMULSIONS COMPRISING SEMIFLUORINATED ALKANES**
O/W-EMULSIONEN MIT SEMIFLUORIERTEN ALKANEN
EMULSIONS H/E COMPRENANT DES ALCANES SEMIFLUORÉS

(30) Priority: 04.01.2011 EP 11150064
(43) Date of publication of application: 13.11.2013
(60) Divisional application: 17159788.3
(73) Proprietor: Novaliq GmbH, 69120 Heidelberg (DE)
(72) Inventor: THEISINGER, Bastian, 68239 Mannheim (DE); THEISINGER, Sonja, 68239 Mannheim (DE); GÜNTHER, Bernhard, 69221 Dossenheim (DE)
(74) Representative: Pharma Concepts GmbH
(86) International application number: PCT/EP2012/050043
(87) International publication number: WO 2012/093113

(56) References cited:
- US-A- 6 113 919

## Description

### BACKGROUND

Pharmaceutical emulsions play a key role in the field of dermatology, where they provide skin-friendly carriers for many topical drugs. Occasionally, emulsions are also used for oral and parenteral medicines, in particular for the intravenous administration of very poorly water-soluble active ingredients such as propofol (marketed e.g. as Disoprivan® or Diprivan®) and etomidate (marketed as Etomidat® Lipuro).

Propofol (2,6-diisopropylphenol, MW 178.27) is potent intravenous anaesthetic. It is routinely used for both the induction and the maintenance of anaesthesia. Moreover, it may be used for sedation of patients in intensive care or in preparation of local or regional anaesthesia for surgical and diagnostic procedures. It is characterised by its rapid onset of action and its relatively moderate side effects.

Physically, propofol is a highly lipophilic compound which melts at about 19 °C. At room temperature, it has the appearance of an oil. Its solubility in water or aqueous buffers is negligible, which makes propofol a highly challenging compound to formulate, in particular for intravenous administration, but also for other routes. The only ionisable group of the molecule is its hydroxyl group, which is however unsuitable for forming a water soluble salt due to its pKa of 11. The octanol/water partition coefficient for propofol is 6761:1 at a pH of 6-8.5.

Propofol was first developed by the British pharmaceutical company ICI (now AstraZeneca) as a solubilised intravenous formulation which contained substantial amounts of the solubiliser Cremophor® EL, an excipient which is not very well tolerated. Shortly after the market introduction, several reports of anaphylactic reactions led to the withdrawal of the formulation. Several years later, AstraZeneca launched a new formulation of propofol branded as Diprivan® which is still used today. This product is an o/w-emulsion comprising 1 % of propofol and 10 % of soy bean oil as the dispersed phase and 1.2 % purified egg lecithin as emulsifier. The coherent aqueous phase contains 2.25 % of glycerol and small amounts of EDTA and sodium hydroxide. In recent years, generic emulsion formulations have also become available in a number of countries.

Propofol is indicated for the induction and maintenance of general anaesthesia, sedation for mechanically ventilated adults, and procedural sedation. Other clinical uses which are still experimental include for the management of status epilepticus, the treatment of headache, in particular migraine headache, the management of anxiety, and neuroprotection in acute brain injury. These uses often require only sub-hypnotic doses of propofol, as taught, for example, in WO 00/54588 A1.

Compared to other compounds used in anaesthesia, propofol has a remarkable safety profile. Its adverse effects are usually mild and easily managed. The hypnotic effect of a single dose of propofol typically wears off within minutes. The rapid onset and recovery along with its amnestic effects have made the compound very popular for sedation and anaesthesia. In contrast to similar agents, it does not appear to induce nausea.

Among the typical adverse effects are a lowered blood pressure and transient apnoea following induction doses. Mild myoclonic movements are commonly observed. Another frequent issue of the propofol emulsion is that it produces local pain at the site of injection or infusion, for which reason some patients are pretreated with a local anaesthetic such as lidocaine. It is believed that the small fraction of propofol dissolved in the aqueous phase of the emulsion is responsible for this pain. Rare but more serious are dystonia, hyperlipidaemia, pancreatitis and the so-called propofol infusion syndrome. This potentially lethal metabolic derangement has occurred in critically ill patients after a prolonged infusion of high-dose propofol in combination with catecholamines and/or corticosteroids.

More recently, other intravenous formulations of propofol have been tested clinically or introduced to the market. For example, a 1 % propofol emulsion with only 5 % of soybean oil and 0.6 % lecithin (Ampofol®) has been studied. It is likely that this formulation may be associated with a lower risk of hyperlipidaemia and pancreatitis. At the same time, the pain at the injection site was found to be even more pronounced than with Diprivan®.

Other emulsion formulations such as Propofol-Lipuro® and IDD-D® propofol rely on a higher fraction of medium chain triglycerides (MCT) to replace long chain triglycerides (LCT) in the oil component of the emulsion. It is assumed that MCT's are better tolerated than LCT's by both adults and paediatric patients. However, they may also release toxic compounds such as acetoacetate, beta-hydroxybutyrate and octanoates.

However, there is still some risk of hyperlipidaemia and pancreatitis involved in the use of propofol emulsions. The relatively low drug load of these emulsions necessitates the administration of substantial amounts of triglycerides and emulsifiers (i.e. phospholipids) having their own specific risk profiles.

A further drawback of parenteral propofol emulsions is that they are, due to their content of triglyceride oil in an aqueous environment and phospholipids as emulsifiers, prone to substantial microbial growth after contamination. Therefore, the present formulation of Diprivan® comprises (di)sodium edetate as an antimicrobial agent, even though the product is restricted to single-patient-use per vial.

While other microbial preservatives for injectable formulations are in principle available, they are associated with a decreased tolerability and in particular with the risk of inducing hypersensitivity reactions. In WO 00/24376, benzyl alcohol alone (0.0175- 0.9 wt.-%) or in combination with sodium edate (0.005 wt.-%) or sodium benzoate (0.07 wt.-%) is used to microbially stabilise an oil-in-water emulsion containing propofol, vegetable oil as solvent, and egg phosphatides as emulsifier.

WO 2007/052288 describes a formulation of a propofol in the form of an oil-in-water emulsion containing triglyceride oils (5-20 % w/v), 1.2 wt.-% natural phosphatides such as purified soy or egg phospholipid, 2.25 wt.-% glycerol as tonicity modifying agent as well as monoglyceryl esters of lauric and capric acid (0.025-0.05 wt.-%), disodium edetate (0.0025-0.001 wt.-%) and/or capric acid (0.025-0.05 wt.-%) as preservative system. This system shows a no more than 10-fold increase in the growth of each Staphylococcus aureus, Eschericha coli, Pseudomonas aeruginose and Candida albicans for at least 24 h.

Alternatively, non-emulsion formulations which have been suggested for propofol include aqueous solutions in which the drug substance is present in solubilised form with the aid of a cyclodextrin. Cyclodextrins are water-soluble cyclic oligosaccharides capable of forming inclusion complexes with guest molecules. In particular, propofol solutions with hydroxypropyl-p-cyclodextrin and with sulphobutylether-β-cyclodextrin, respectively, have been studied. However, it has not been established whether the pharmacokinetics of these formulations is comparable to the propofol emulsions. Moreover, high doses of cyclodextrins are often linked with haemolytic effects and renal toxicity.

Thus there remains a need for further improved formulations of poorly water-soluble compounds such as propofol. Moreover, there is a need for improvements in the formulation of pharmaceutical emulsions. For example, there is a need for emulsions having a higher drug load and/or a better safety profile.

US patent no. 6,113,919 describes oil-in-water emulsions comprising an aqueous continuous phase and an oily dispersed phase, wherein the dispersed phase comprises at least two fluorinated liquids, one of which is the oily solvent or carrier and constitutes the bulk of the dispersed phase, the second one being a fluorinated co-surfactant at an amount of up to 10% w/v of the dispersed phase. The fluorinated co-surfactant is different from the fluorinated oily carrier. The oily carrier is a perfluorinated liquid, in particular perfluorooctyl bromide, which is used in amounts of about 90% w/v relative to the dispersed phase.

However, perfluorinated compounds are rather problematic in emulsions, in particular if they are incorporated in high amounts or used as the carrier or solvent of the oil phase. For example, their density is extremely high so that there is always a risk of physical phase separation through sedimentation of the dispersed phase, which is difficult to control. Usually, large amounts of surfactant are necessary to stabilise an emulsion based on a perfluorocarbon. These emulsions are typically sensitive to mechanical stress such as associated with pumping, dispensing, centrifigation etc. Moreover, perfluorinated compounds are extremely lipophobic and hydrophobic and thus not very suitable as solvents for many active ingredients that would require at least a more moderate degree of lipophilicity and/or hydrophilicity.

It is therefore an object of the present invention to provide such improved compositions which overcome one or more disadvantages of known compositions. In particular, it is an object of the invention to provide propofol compositions which exhibit a high drug content and/or a high microbial stability. Another object is to provide propofol compositions which are not associated with the risk of hyperlipidaemia or pancreatitis. Further objects of the invention will become clear on the basis of the description of the invention below, including the examples, and of the patent claims.

### SUMMARY OF THE INVENTION

The invention provides a novel composition in the form of a liquid, physically stable O/W-emulsion comprising (a) a dispersed phase comprising a semifluorinated alkane according to formula RFRH, (b) an aqueous continuous phase, and (c) at least one surfactant, wherein RF is a linear perfluorinated hydrocarbon segment with 4 to 12 carbon atoms, and RH is a linear alkyl group with 4 to 8 carbon atoms, and wherein perfluorinated compounds are absent in the dispersed phase. The composition is further characterised in that the average droplet size of the dispersed phase is below about 1 µm.

In one of the preferred embodiments, the dispersed phase of the composition comprises a poorly water-soluble active pharmaceutical agent such as propofol. Preferably, the propofol emulsion exhibits a high drug load, i.e. the propofol concentration in the dispersed phase is at least about 10 wt.-%. Preferably, the propofol composition is sterile. Due to the high antimicrobial stability of semifluorinated alkanes, the composition may be free of preservatives. It is also preferably free of triglyceride oils and therefore not associated with the risk of hyperlipidaemia and pancreatitis.

In a further aspect, the invention provides a liquid O/W-emulsion which is highly concentrated in that its dispersed phase represents at least about 50 wt.-% of the emulsion. Such highly concentrated versions are particularly suitable for being used as preconcentrates which can be diluted with specific aqueous liquids prior to use. Moreover, they are useful for accommodating high amounts of water-insoluble drug substances or other hydrophobic active agents. Such emulsions may also be used for topical (e.g. dermal) administration.

Further aspects of the invention related to the uses of such O/W-emulsions comprising semifluorinated alkanes. For example, they may be used pharmaceutically, e.g. as therapeutic or diagnostic preparations. As such, they may be administered topically, orally, or parenterally. In particular, emulsions comprising propofol may be used for general anaesthesia and/or sedation, and may be administered by intravenous injection or infusion. Emulsions comprising an active agent useful for the preservation of organ or tissue transplants, such as N-octanoyl dopamine, may be administered to an organ or tissue transplant donor, to an organ or tissue transplant recipient, and/or for the flushing or immersion of an organ or tissue transplant for storage or transport.

Furthermore, the compositions of the invention may be used as cosmetic preparations or as veterinary medicaments.

### BRIEF DESCRIPTION OF THE DRAWINGS

Figure 1 shows the results of a test for haemolytic activity involving various propofol-comprising compositions according to the invention and of a propofol emulsion which is not according to the invention (ST174). Details are discussed in example 14.
Figure 2 shows the results of a test for haemolytic activity in the presence of human serum, as described in more detail in example 14.
Figure 3 shows the sedation effectiveness of a propofol emulsion according to the invention in comparison with a conventional propofol product determined in a rat model, as described in example 15.
Figure 4 shows the propofol plasma profiles in rats after intravenous bolus administration of a propofol emulsion according to the invention in comparison with a conventional propofol product, as described in example 16.

### DETAILED DESCRIPTION OF THE INVENTION

The invention provides a novel composition in the form of a liquid, physically stable O/W-emulsion comprising (a) a dispersed phase comprising a semifluorinated alkane according to formula RFRH, (b) an aqueous continuous phase, and (c) at least one surfactant, wherein RF is a linear perfluorinated hydrocarbon segment with 4 to 12 carbon atoms, and RH is a linear alkyl group with 4 to 8 carbon atoms, and wherein perfluorinated compounds are absent in the dispersed phase. The composition is further characterised in that the average droplet size of the dispersed phase is below about 1 µm.

As used herein, an emulsion is a liquid system comprising a dispersed (or inner, or emulsified, or discontinuous) liquid phase within a continuous (or outer, or coherent) liquid phase. The two liquid phases are not miscible. In an O/W-emulsion (also referred to as oil-in-water emulsion), a water-immiscible organic liquid phase, which does not have to be an "oil" by any specific definition, is dispersed in a water-miscible continuous phase which may or may not be substantially comprised of water itself.

Semifluorinated alkanes are linear or branched alkanes some of whose hydrogen atoms have been replaced by fluorine. The semifluorinated alkanes (SFA's) used in the present invention are composed of one non-fluorinated hydrocarbon segment and one perfluorinated hydrocarbon segment. The SFA used in the present invention have one non-fluorinated hydrocarbon segment attached to one perfluorinated hydrocarbon segment, according to the general formula F(CF₂)ₙ(CH₂)ₘH.

Another nomenclature which is used herein refers to the above-mentioned SFA's having two segments as RFRH, respectively, wherein R_{F} designates a perfluorinated hydrocarbon segment, R_{H} designates a non-fluorinated segment. Alternatively, the compounds may be referred to as FnHm, wherein F means a perfluorinated hydrocarbon segment, H means a non-fluorinated segment, and n and m is the number of carbon atoms of the respective segment. For example, F3H3 is used for perfluoropropylpropane. Moreover, this type of nomenclature is usually used for compounds having linear segments. Therefore, unless otherwise indicated, it should be assumed that F3H3 means 1-perfluoropropylpropane, rather than 2-perfluoropropylpropane, 1-perfluoroisopropylpropane or 2-perfluoroisopropylpropane.

Semifluorinated alkanes according to the general formula F(CF₂)ₙ(CH₂)ₘH may have segment sizes ranging from 3 to 20 carbon atoms, i.e. n and m are independently selected in the range from 3 to 20. SFA's which are useful in the context of the present invention are also described in EP-A 965 334, EP-A 965329 and EP-A 2110126.

In the present invention, the semifluorinated alkane is a compound according to the formula RFRH, whose segments R_{F} and R_{H} are linear; the perfluorinated segment is linear and comprises from 4 to 12 carbon atoms, and the non-fluorinated segment is linear and comprises from 4 to 8 carbon atoms. Preferred SFA's include in particular the compounds F4H5, F4H6, F4H8, F6H4, F6H6 and F6H8. Presently most preferred for carrying out the invention are F4H5, F4H6, F4H8, F6H6 and F6H8.

Optionally, the dispersed phase of the emulsion may comprise more than one SFA. It may be useful to combine SFA's, for example, in order to achieve a particular target property such as a certain density, viscosity, or solubilising capacity for a particular active ingredient. If a mixture of SFA's is used, it is furthermore preferred that the mixture comprises at least one of F4H5, F4H6, F6H4, F6H6 and F6H8, and in particular one of F4H5, F4H6, F6H6 and F6H8. In another embodiment, the mixture comprises at least two members selected from F4H5, F4H6, F6H4, F6H6 and F6H8, and in particular at least two members selected from F4H5, F6H6 and F6H8. Perfluorinated compounds are absent.

Liquid SFA's are chemically and physiologically inert, colourless and stable. Their typical densities range from 1.1 to 1.7 g/cm³, and their surface tension may be as low as 19 mN/m. SFA's of the RFRH type are insoluble in water but also somewhat amphiphilic, with increasing lipophilicity correlating with an increasing size of the non-fluorinated segment. Again, for practising the current invention, an SFA having a density of at least 1.2 g/cm³ should be selected.

Liquid SFA's of the RFRH type are being used commercially for unfolding and reapplying a retina, for long-term tamponade as vitreous humor substitute (H. Meinert et al., European Journal of Ophthalmology, Vol. 10(3), pp. 189-197, 2000), and as wash-out solutions for residual silicon oil after vitreo-retinal surgery. Experimentally, they have also been used as blood substitutes (H. Meinert et al., Biomaterials, Artificial Cells, and Immobilization Biotechnology, Vol. 21(5), pp. 583-95, 1993). These applications have established SFA's as physiologically well tolerated compounds. On the other hand, SFA's have not been used as excipients in approved drug products as of today.

The dispersed phase of the emulsion may comprise other constituents, such as one or more organic diluents (for example, triglyceride oils), osmotic stabilisers, colouring agents, antioxidants (for example, α-tocopherol), or another compound potentially useful in view of the intended product use or the incorporated active ingredient, if any. On the other hand, the dispersed phase comprises no perfluorinated compound such as perfluorooctyl bromide or perfluorodecalin.

The composition further comprises a surfactant. While semifluorinated alkanes exhibit some degree of amphiphilicity, the composition of the invention requires the presence of a surfactant which is not a semifluorinated alkane. The surfactant is selected and incorporated at an amount capable of physically stabilising the emulsion. It is believed that the surfactant is present at the interface of the dispersed liquid droplets and the continuous phase and, optionally, in the continuous phase itself.

Preferably, the surfactant is physiologically acceptable in view of the intended purpose and route of administration. In one of the preferred embodiments, at least one surfactant exhibiting an HLB-value of 8 or higher is incorporated. In a further embodiment, an incorporated surfactant exhibits an HLB-value of about 12 or higher, or about 14 or higher. As used herein, the HLB-value refers to the hydrophilic-lipophilic balance commonly used to describe the degree to which an amphiphilic molecules such as a surfactant is hydrophilic or lipophilic. The HLB value may be calculated on the basis of the relative size of the different regions of the molecule, as originally proposed by W. Griffin (Classification of Surface-Active Agents by 'HLB'; Journal of the Society of Cosmetic Chemists 1, 311, 1949).

The surfactant may be ionic or nonionic. In a particular embodiment, the composition comprises an ionic surfactant preferably selected from the class of phospholipids. Phospholipids are surfactants composed of a phosphate group (imparting a negative charge) which is, on one side, linked to a small basic residue (usually imparting a positive charge) such as choline or ethanolamine, and on the other side to glycerol or sphingosine. The glycerol residue is esterified with two fatty acid residues which represent the lipophilic part of most of the phospholipid molecules.

Among the preferred phospholipids are native, hydrated and/or purified lecithins, such as lecithin derived from eggs or soy beans, typically comprising high amounts of phosphatidylcholines. Also preferred are native, purified, synthetic or semisynthetic phosphatidylcholines, either having mixed fatty acid residues as found in their native sources or generated by hydration; or specific fatty acid compositions as in the case of e.g. dimyristoyl phosphatidylcholine, dipalmitoyl phosphatidylcholine, and distearoyl phosphatidylcholine. Furthermore, phospholipid extracted from animal organs such as lungs may be used, for example pulmonary phospholipids from pigs, as comprised in the product, Curosurf®. Particularly preferred surfactants are purified and optionally hydrated lecithins extracted from eggs or soy beans.

In an alternative preferred embodiment, the surfactant is selected from the class of physiologically acceptable nonionic surfactants. Examples for such surfactants include in particular:
- pegylated glycerides such as macrogol-15-hydroxystearate (e.g. Solutol® HS 15), macrogol glycerol ricinoleate-35 (e.g. Cremophor® EL), macrogol glycerol hydroxystearate-40 (e.g. Cremophor® RH 40), macrogol-1000-glycerol monolaurate, macrogol-1000-glycerol monostearate, and macrogol-1000-glycerol monooleate;
- pegylated fatty acids such as macrogol stearate 400, polyoxyl 40 stearate, and polyoxyl 60 stearate;
- pegylated fatty alcohols such as macrogol laurylether, polyoxyl 20 cetostearylether, and polyoxyl 10 oleylether;
- pegylated sorbitan fatty acid esters such as polysorbate 20, polysorbate 40, polysorbate 60, and polysorbate 80 (e.g. Tween® 20/40/60/80); and
- triblock copolymers of polyoxyethylene and polyoxypropylene, such as poloxamer 124, poloxamer 188, poloxamer 237, poloxamer 338, and poloxamer 407. From the above, polysorbates are particularly preferred.

It is also possible to incorporate more than one surfactant within the composition of the invention. For example, the combination of an ionic surfactant such as a lecithin and a nonionic surfactant such as a polysorbate or poloxamer may be useful for stabilising the emulsion in case that the continuous phase comprises substantial amounts of salts or other electrolytes.

In a further embodiment, a non-ionic surfactant is selected as a sole surfactant in a composition comprising sodium chloride or another salt in the continuous phase of the emulsion. In another embodiment, an ionic surfactant such as a lecithin or phosphatidylcholine is selected as the sole surfactant, and the continuous phase of the emulsion is substantially free of salts such as sodium chloride. If an osmotic agent is required in this case, such agent is preferably selected from nonionic, physiologically acceptable osmotic agents such as sugars (e.g. glucose) or sugar alcohols (such as mannitol or sorbitol).

It has been discovered by the inventors that, on the basis of the guidance provided above with respect to the selection of components of the dispersed phase, the continuous phase and the surfactant, emulsion systems can be prepared by common emulsification systems which are physically stable. In this context, physically stable means stable against major changes in the droplet size distribution of the emulsion, and in particular against coalescence. Preferably, the physical stability includes resistance to substantial particle size growth even under heating, e.g. to temperatures at which the emulsion is pasteurised or even sterilised. Contrary to the teachings of prior art, the inventors have thus found O/W-emulsions comprising semifluorinated alkanes which are heat sterilisable even in the presence of salts. As used herein, substantial particle size growth is understood as a growth of the average droplet size of the emulsion from a starting value to more than about 150% of that value.

The average droplet size of the composition of the invention is understood as the z-average droplet size as measure by laser diffraction or dynamic light scattering. It is less than about 1 µm, but depending on the specific product application, it may also be selected to be less than about 500 nm, as is preferred in case the composition is intended for intravenous injection or infusion. According to further embodiments, the average droplet size is up to about 400 nm, or up to about 300 nm, or from about 150 nm to about 400 nm, respectively.

Emulsions based on semifluorinated alkanes exhibiting such average droplet sizes can be prepared by generally known techniques with or without adaptation. For example, the components of the emulsion may be combined and emulsified by high-sheer homogenisation, high pressure homogenisation, ultrasonic homogenisation, static mixing, and the like. It is recommendable to first prepare, separately, the hydrophilic and lipophilic solutions which will constitute the dispersed and continuous phases of the O/W-emulsion, respectively. The surfactant(s) and, optionally, any further hydrophilic additives may be dissolved in a suitable vehicle for the continuous phase, such as water or aqueous buffer. Similarly, the components for the dispersed phase, i.e. the selected semifluorinated alkane(s), along with any further optional compound such as a lipophilic or hydrophobic drug substance, may be provided as a solution. Subsequently, the two solutions may be mixed and homogenised until the desired droplet size distribution is obtained. Optionally, the mixture is cooled during homogenisation.

The emulsion may be filled into vials or any other suitable vessels. If a sterile emulsion is required, the emulsion may be prepared from sterile constituents by aseptic processing. Alternatively, the emulsion may be sterilised by filtration through an appropriate filter, having e.g. a pore size of about 0.2 µm. In one of the preferred embodiments, the emulsion is sterilised by heat, for example by applying a pasteurisation process, and more preferably by autoclaving at e.g. 121 °C. It is one of the particular advantages of the present invention that it provides emulsions which are physically stable not only at room temperature, but also at elevated temperatures such as during autoclaving. Indeed, according to one of the preferred embodiments, the composition of the invention is sterile, or heat sterilisable.

While sterility is an important requirement in the context of certain product applications, in particular for products for parenteral or ophthalmic administration, it may also be of great importance to ensure that the product does not lose its microbial purity during use. For example, the US Food and Drug Administration (FDA) and Center for Disease Control (CDC) require that any microbial growth in the time period of 24 hours after opening a sterile vessel for immediate parenteral use of the content is less than 10-fold. Unexpectedly, it has been found by the inventors that emulsions as provided by the invention do not support microbial growth as, for example, conventional emulsions comprising triglyceride oils do. The resistance to microbial growth is even pronounced in cases in which a lecithin (which also tends to support microbial growth) is used as emulsifier. Accordingly, in one of the preferred embodiments, the composition of the invention is substantially free of any preservatives. In another embodiment, the composition comprises a small amount of preservative, but at a level which would not prevent microbial growth in the absence of a semifluorinated alkane.

The content of dispersed phase in the composition will depend on the intended product use. For example, it may range from about 1 to about 80 wt.-%. The excellent physiological tolerability of semifluorinated alkanes and the high physical stability achieved by the emulsions of the invention allow the preparation and use of compositions with surprisingly high amounts of dispersed phase, in particular 50 wt.-% and more, or even higher than 60 wt.-%, such as from about 60 wt.-% to about 80 wt.-%. Such highly concentrated emulsions are particularly suitable for being used as preconcentrates which can be diluted with specific aqueous liquids prior to use. Moreover, they are useful for accommodating high amounts of water-insoluble drug substances or other hydrophobic active agents. Such emulsions may also be used for topical (e.g. dermal) administration.

The present invention is particularly useful as a human or veterinary medicine. The dispersed phase of the emulsion based on a semifluorinated alkane is a highly suitable carrier for poorly water-soluble, or hydrophobic, or lipophilic, active pharmaceutical ingredients. In one of the preferred embodiments, the composition comprises at least one such active ingredient in dissolved form within the dispersed phase.

As used herein, an active pharmaceutical ingredient is a compound or mixture of compounds useful in the diagnosis, prevention, management, and/or therapy of a human or animal disease or condition. It may also be referred to as active ingredient, active agent, active compound, drug substance, and the like.

Again, the active ingredient incorporated within the composition is preferably poorly water-soluble. In particular, it water solubility is preferably not more than about 1 mg/mL. In other preferred embodiments, the water solubility is not higher than about 0.1 mg/mL, or not more than about 10 µg/mL, respectively. The invention is particularly useful for delivering such active ingredients because it allows the administration of effective doses in relatively small volumes, which is partly due to the surprisingly high solubilisation capacity of semifluorinated alkanes for many poorly water-soluble drug substances, but also a beneficial effect of the high physical stability of the emulsions which allow the formulation of compositions comprising large amounts of dispersed phase.

In a specific embodiment, the composition comprises a poorly water-soluble active ingredient selected from the class of general anaesthetic agents, which includes candidate compounds such as propofol and etomidate. Particularly preferred is propofol, also known as 2,6-diisopropylphenol, whose properties and uses have already been described above.

The inventors have surprisingly discovered that semifluorinated alkanes have a remarkable capability to solubilise propofol. In fact, propofol is fully miscible with certain highly preferred semifluorinated alkanes, such as F4H5, F4H6 and F6H8, over a wide temperature range, in others it exhibits very high solubility. While it was previously suggested that semifluorinated alkanes could be useful solvents for certain lipophilic compounds, this extraordinary level of solubilisation capacity could not have been predicted for propofol. By virtue of the high solubility of propofol in semifluorinated alkanes, it is now possible to provide injectable propofol formulations having a high strength (or concentration of propofol), and thus require only a low volume of administration. This will also bring about an improved tolerability, since a lower administration volume inherently means a lower intake of excipients (such as emulsifiers). In addition, these compositions are free of triglyceride oils and therefore do not exhibit the disadvantages associated with triglycerides, including the risk of hyperlipidaemia and pancreatitis.

Preferred semifluorinated alkanes for formulating propofol emulsions according to the invention include in particular the compounds F4H5, F4H6, F6H4, F6H6 and F6H8. Presently most preferred are F4H5, F4H6 and F6H8. Optionally, the composition may comprise more than one SFA. It may be useful to combine SFA's, for example, in order to achieve a particular target property such as a certain density or viscosity. If a mixture of SFA's is used, it is furthermore preferred that the mixture comprises at least one of F4H5, F4H6, F6H4, F6H6 and F6H8, and in particular one of F4H5, F4H6 and F6H8. In another embodiment, the mixture comprises at least two members selected from F4H5, F4H6, F6H4, F6H6 and F6H8" and in particular at least two members selected from F4H5, F4H6 and F6H8.

In order to enable safe and convenient dosing and administration, the composition of the invention should have a strength (i.e. concentration of propofol) in the range from about 0.1 wt-% to about 50 wt.-%. In further embodiments, the strength is about 1 wt.-% or higher, such as at least about 2 wt.-%, 5 wt.-%, 10 wt.-% or 20 wt.-%. The propofol concentration in the dispersed phase may range from 1 wt.-% up to 99 wt.-%. In specific embodiments, the dispersed phase contains about 10-50 wt.-% propofol dissolved in semifluorinated alkane(s), such as about 10 wt.-%, 20 wt.-%., 30 wt.-%, 40 wt.-%, or 50 wt.-%, respectively.

The emulsifier is preferably a compound with proven safety for parenteral use. In certain embodiments, the emulsifier is selected as described above in the context of semifluorinated alkane-based emulsions according to the invention in general. In other specific embodiments, the emulsifier is selected from lecithins, phosphatidylcholines, polysorbates, and poloxamers. The amount of surfactant is selected in consideration of the amount of the dispersed phase in the emulsion. It may range, for example, from about 0.5 wt.-% to about 100 wt.-% relative to the weight of the dispersed phase. In further embodiments, the surfactant is present at an amount ranging from about 2 wt.-% to about 50 wt.-% relative to the dispersed phase, or from about 2 wt.-% to about 20 wt.-%, or from about 4 wt.-% to about 10 wt.-%, respectively. In the case that more than one surfactant is used, the percentages relate to the total surfactant quantity.

The propofol emulsions may contain further excipients, in particular in the continuous phase which is preferably based on water. For example, it is preferred that an excipient or excipient mixture is incorporated in order to ensure that the osmotic pressure of the composition is in the physiologically acceptable range, such as in the range from about 200 to about 500 mOsmol/kg, or more preferably in the range from about 250 to about 400 mOsmol/kg, or from about 280 to about 350 mOsmol/kg, respectively. The osmolality may be adjusted with commonly used excipients that are physiologically acceptable, such as sodium chloride, sugars like glucose, sugar alcohols like mannitol or sorbitol, and the like. In case sodium chloride or other salts are used for this purpose, the surfactant or, if more than one surfactant is used, at least one of the surfactants should be selected from the class of nonionic surfactants.

In a further preferred embodiment, one or more excipients are incorporated to keep the pH of the propofol composition within a physiologically acceptable range. In particular, the pH of the composition may be adjusted to pH 4 to pH 9, or more preferably within the range from pH 5 to pH 8, such as from pH 6 to pH 7.5. For adjusting and, optionally, buffering the pH value, physiologically acceptable acids, bases, salts, and combinations of these may be used. Suitable excipients for lowering the pH value or as acidic components of a buffer system are strong mineral acids, in particular, sulphuric acid and hydrochloric acid. Moreover, inorganic and organic acids of medium strength as well as acidic salts may be used, for example, phosphoric acid, citric acid, tartaric acid, succinic acid, fumaric acid, methionine, acidic hydrogen phosphates with sodium or potassium, lactic acid, glucuronic acid etc. However, sulphuric acid and hydrochloric acid are most preferred. Suitable for raising the pH value or as basic component for buffer system are, in particular, mineral bases such as sodium hydroxide or other alkali and alkaline earth hydroxides and oxides such as, in particular, magnesium hydroxide and calcium hydroxide, ammonium hydroxide and basic ammonium salts such as ammonium acetate, as well as basic amino acids such as lysine, carbonates such as sodium or magnesium carbonate, sodium hydrogen carbonate, citrates such as sodium citrate etc. Moreover, readily available buffered, isotonic aqueous solutions may be used as a basis for the preparation of the continuous phase.

The propofol emulsions according to the invention may be used in the same manner and for the same purposes as conventional propofol compositions, i.e. for the induction and maintenance of general anaesthesia, or for sedation. The compositions are preferably provided in sterile form and injected or infused intravenously.

In a further embodiment, the composition of the invention is provided as an emulsion having a high content of dispersed phase, such as about 40 wt.-% or more, or about 50 wt.-% or more, or preferably at least about 60 wt.-%, or from about 60 wt.-% to less than about 80 wt.-%, respectively, and adapted for use as a preconcentrate for being diluted with an aqueous organ preservation solution such as HTK (histidine-tryptophan-ketoglutarate) solution or UW (University of Wisconsin) solution. Semifluorinated alkanes exhibit a high capacity to dissolve and carry oxygen, which makes these compounds highly attractive for organ or tissue preservation. While semifluorinated alkanes per se are not miscible with conventional aqueous organ preservation solutions, the emulsions provided by the invention are physically highly stable and compatible with such solutions. They can be easily and conveniently diluted with these prior to use such as to combine the beneficial effects of semifluorinated alkanes with those of conventional organ preservation solutions. Of course, it is also possible to incorporate an active pharmaceutical ingredient within a concentrated emulsion intended for organ or tissue preservation.

Such composition is preferably sterile and adapted for parenteral administration. It may be administered, in particular after dilution, systemically or locally to a transplant donor for pre-treatment and prevention of ischaemic damage, or used in vitro to flush and preserve allografts in order to minimise cold preservation injury.

Other active ingredients and product applications are also contemplated. For example, the composition may comprise a poorly water-soluble active ingredient useful for the prevention or therapy of a disease or condition of the skin or a mucosa, and adapted for topical, e.g. dermal or mucosal (including buccal and sublingual), administration. Even without the incorporation of any particular active pharmaceutical ingredient, the composition may be used for cosmetic purposes as it was found to exhibit an excellent tolerability with respect to the skin and a good spreading behaviour.

The invention is further illustrated by the following examples.

### Examples

### Example 1

10 g of propofol were dissolved in 10 g of F6H8. This mixture was added to a solution of 800 mg S75 (soy bean lecithin, Lipoid AG) in 979.2 g of aqueous dextrose solution (5 wt.-%) in water and stirred for 1 h at 2000 rpm. The emulsion was then prepared by high pressure homogenization process using an Avestin C3 apparatus at a pressure of 1100 bar in continuous process for 1 hour. The pH value was adjusted to pH 7,3-7,5 by adding sodium hydroxide solution. The final emulsion was filled into vials, closed and sealed after blanketing with nitrogen. Subsequently, the vials were sterilised at 121 °C for 10 minutes.

The average droplet size was 212 nm. Surprisingly, during the first 6 month of storage at 23 °C, the mean droplet size showed no significant increase. From this batch, 20 vials was tested according to Ph. Eur. 6 and found to be sterile.

### Example 2

0.1 g propofol was dissolved in 0. 1 g of F6H8. This mixture was added to a solution of 16 mg of S75 in 9.784 g of dextrose solution (5 wt.-%) in water and stirred for 1 h at 2000 rpm. The emulsion was formed by ultrasonicating the pre-emulsion for 240 s (Is pulse, Is break) at 100 % amplitude (Hilcher sonifier, 1/4 inch tip) under ice-cooling. The pH value was adjusted to pH 7.3-7.5 by adding sodium hydroxide solution. The final emulsion was filled into vials, closed and sealed after blanketing with nitrogen. Subsequently, the vials were sterilised at 121 °C for 10 minutes.

### Example 3

In essentially the same manner as in example 2, a sterilised emulsion was prepared from 0.1 g of propofol, 0.5 g of F6H8, and a solution of 80 mg of S75 in 9.72 g of dextrose solution (5 wt.-%).

### Example 4

In essentially the same manner as in example 2, a sterilised emulsion was prepared from 0.2 g of propofol, 0.2 g of F6H8, and a solution of 32 mg of S75 in 9.568 g of dextrose solution (5 wt.-%) containing HEPES (10 mmol/L).

### Example 5

In essentially the same manner as in example 2, a sterilised emulsion was prepared from 1 g of propofol, 1 g of F6H8, and a solution of 160 mg of S75 in 7.84 g of dextrose solution (5 wt.-%).

### Example 6

In essentially the same manner as in example 2, a sterilised emulsion was prepared from 0.1 g of propofol, 0.3 g of F6H8, and a solution of 16 mg of S75 in 9.784 g of dextrose solution (5 wt.-%).

### Example 7

In essentially the same manner as in example 2, a sterilised emulsion was prepared from 0.1 g of propofol, 0.8 g of F6H8, 0.2 g of olive oil (Sigma Aldrich) and a solution of 8 mg of S75 in 9.792 g of dextrose solution (5 wt.-%).

### Example 8

In essentially the same manner as in example 2, a sterilised emulsion was prepared from 0.1 g of propofol, 0.1 g of F6H8, and a solution of 8 mg of S75 and 8 mg of sodium oleate in 9.784 g of dextrose solution (5 wt.-%).

### Example 9

In essentially the same manner as in example 2, a sterilised emulsion was prepared from 0.2 g of propofol, 0.2 g of F6H8, and a solution of 32 mg of S75 and 8 mg of sodium oleate in 9.568 g of dextrose solution (5 wt.-%).

### Example 10

In essentially the same manner as in example 2, a sterilised emulsion was prepared from 0.1 g of propofol, 0.067 g of F6H8, 0.033 g of F4H5, and a solution of 16 mg of EPCS (Lipoid AG) in 9.784 g of dextrose solution (5 wt.-%).

### Example 11

In essentially the same manner as in example 1 except that, additionally, 3 mg of the dye Patent Blue V was used, a sterilised blue emulsion was prepared.

### Example 12

In essentially the same manner as in example 2, a sterilised emulsion was prepared from 0.1 g of propofol, 0.01 g of α-tocopherol, 0.1 g of F6H8, and a solution of 8 mg of S75 in 9.792 g of dextrose solution (5 wt.-%).

### Example 13

An antimicrobial preservative effectiveness test in analogy to that of USP 32 <51> was carried out. Sample vials prepared according to Examples 1, 3 and 4 were inoculated with *Escherichia coli, Pseudomonas aeruginosa, Staphylococcus aureus, Candida albicans* and *Aspergillus niger* and incubated at approx. 22.5 °C. Commercially obtained samples of Disoprivan® were tested as comparators. The results are given in tables 1 to 4.

Surprisingly, no increase but a substantial decrease in the concentration of colony-forming units (cfu) over 24 and 48 hours was observed in the case of emulsions comprising semifluorinated alkanes. Obviously, the emulsions according to the invention do not support microbial growth, but rather inhibit it in the same manner as if an effective amount of an antimicrobial preservative had been added. In contrast, the Disoprivan® samples showed a marked increase in cfu/mL for *Escherichia coli, Staphylococcus aureus und Candida albicans.*

**Table 1**

| **Emulsion of example 1** | **cfu/mL** | | | **Change in log steps** | |
|---|---|---|---|---|---|
| **Organism** | **0 h** | **24h** | **48h** | **24 h** | **48 h** |
| E. coli (ATCC 8739) | 880 | <100 | <100 | ≤-0.9 | ≤-0.9 |
| P. aeruginosa (ATCC 9027) | 910 | <100 | <100 | ≤-0.9 | ≤-0.9 |
| S. aureus (ATCC 6538) | 940 | <100 | <100 | ≤-0.9 | ≤-0.9 |
| C. albicans (ATCC 10231) | 950 | <100 | <100 | ≤-0.9 | ≤-0.9 |
| A. niger (ATCC 16404) | 760 | 100 | 100 | -0.9 | -0.9 |

**Table 2**

| **Disoprivan®** | **cfu/mL** | | | **Change in log steps** | |
|---|---|---|---|---|---|
| **Organism** | **0 h** | **24h** | **48h** | **24 h** | **48 h** |
| E. coli (ATCC 8739) | 880 | 1 000 000 | > 1 000 000 | +3.0 | ≥3.0 |
| P. aeruginosa (ATCC 9027) | 910 | <100 | 200 | ≤-0.9 | -0.75 |
| S. aureus (ATCC 6538) | 940 | 85 000 | > 1 000 000 | +2 | ≥3.0 |
| C. albicans (ATCC 10231) | 950 | 14 000 | 210 000 | +1.2 | +2.4 |
| A. niger (ATCC 16404) | 760 | 2 000 | 700 | +0.4 | ±0.0 |

**Table 3**

| **Emulsion of example 3** | **cfu/mL** | | | **Change in log steps** | |
|---|---|---|---|---|---|
| **Organism** | **0 h** | **24h** | **48h** | **24 h** | **48 h** |
| E. coli (ATCC 8739) | 3 000 | <100 | <100 | ≤-1,4 | ≤-1.4 |
| P. aeruginosa (ATCC 9027) | 2 700 | <100 | <100 | ≤-1,4 | ≤-1.4 |
| S. aureus (ATCC 6538) | 2 500 | <100 | <100 | ≤-1,3 | ≤-1.3 |
| C. albicans (ATCC 10231) | 2 500 | <100 | <100 | ≤-1,3 | ≤-1.3 |
| A. niger (ATCC 16404) | 2 500 | 2 600 | 1 950 | ±0,0 | -0.1 |

**Table 4**

| **Emulsion of example 4** | **cfu/mL** | | | **Change in log steps** | |
|---|---|---|---|---|---|
| **Organism** | **0 h** | **24h** | **48h** | **24 h** | **48 h** |
| E. coli (ATCC 8739) | 1 400 | <100 | <100 | ≤-1.1 | ≤-11 |
| P. aeruginosa (ATCC 9027) | 1 400 | <100 | <100 | ≤-11 | ≤-11 |
| S. aureus (ATCC 6538) | 1 300 | <100 | <100 | ≤-11 | ≤-11 |
| C. albicans (ATCC 10231) | 1400 | <100 | <100 | ≤-11 | ≤-11 |
| A. niger (ATCC 16404) | 1600 | <100 | <100 | ≤-1.2 | ≤-1.2 |

To determine whether the observed inhibition of microbial growth in the emulsions of the invention is an effect of the semifluorinated alkanes or the propofol, the test was repeated with a placebo emulsion containing no propofol, but only F6H8 in a higher concentration (40 wt.-%) and S75 in dextrose solution. Also tested were pure solutions of F6H8 and F4H5. The results are given in table 5 to 7 and indicate that this propofol-free emulsion is also capable of reducing the concentration of *Escherichia coli, Pseudomonas aeruginosa* and *Staphylococcus aureus.* In case of *Aspergillus niger,* an initial decrease in cfu concentration was obtained, but after 48 hours it was back to the starting level. Only *Candida albicans* concentrations increased substantially, probably due to the presence of lecithin.

**Table 5**

| **F6H8 placebo emulsion** | **cfu/mL** | | | **Change in log steps** | |
|---|---|---|---|---|---|
| **Organism** | **0 h** | **24h** | **48h** | **24 h** | **48 h** |
| E. coli (ATCC 8739) | 1 400 | 600 | 300 | -0.3 | -0.6 |
| P. aeruginosa (ATCC 9027) | 1 400 | <100 | <100 | ≤-11 | ≤-11 |
| S. aureus (ATCC 6538) | 1 300 | 300 | 100 | -0.6 | -1.1 |
| C. albicans (ATCC 10231) | 1400 | 2 100 | < 1 000 000 | +0.1 | ≥2.8 |
| A. niger (ATCC 16404) | 1 400 | 400 | 1400 | -0.5 | ±0.0 |

**Table 6**

| **F6H8 (pure)** | **cfu/mL** | | | | **Change in log steps** | | |
|---|---|---|---|---|---|---|---|
| **Organism** | **0 h** | **24h** | **48h** | **6d** | **24 h** | **48 h** | **6d** |
| E. coli (ATCC 8739) | 1 600 | <100 | <100 | n.d. | ≤-1.2 | ≤-1.2 | n.d. |
| P. aeruginosa (ATCC 9027) | 1 600 | <100 | <100 | n.d. | ≤-1.2 | ≤-1.2 | n.d. |
| S. aureus (ATCC 6538) | 2 900 | <100 | <100 | n.d. | ≤-1.4 | ≤-1.4 | n.d. |
| C. albicans (ATCC 10231) | 1 500 | <100 | <100 | n.d. | ≤-1.2 | ≤-11 | n.d. |
| A. niger (ATCC 16404) | 1 500 | <100 | <100 | <100 | ≤-1.2 | ≤-1.2 | ≤-1.2 |

**Table 7**

| **F4H5 (pure)** | **cfu/mL** | | | | **Change in log steps** | | |
|---|---|---|---|---|---|---|---|
| **Organism** | **0 h** | **24h** | **48h** | **6d** | **24 h** | **48 h** | **6d** |
| E. coli (ATCC 8739) | 1 600 | <100 | <100 | n.d. | ≤-1.2 | ≤-1.2 | n.d. |
| P. aeruginosa (ATCC 9027) | 1 600 | <100 | <100 | n.d. | ≤-1.2 | ≤-1.2 | n.d. |
| S. aureus (ATCC 6538) | 2 900 | <100 | <100 | n.d. | ≤-1.4 | ≤-1.4 | n.d. |
| C. albicans (ATCC 10231) | 1 500 | <100 | <100 | n.d. | ≤-1.2 | ≤-11 | n.d. |
| A. niger (ATCC 16404) | 3 000 | <100 | <100 | <100 | ≤-1.4 | ≤-1.2 | ≤-1.2 |

### Example 14

Emulsions were prepared essentially as in example 2 end tested for their haemolytic effects on human erythrocytes in the presence and absence of human serum. The continuous phase of the emulsions consisted of aqueous glucose solution (5 wt.-%). The other constituents of the emulsions are given in table 8. In short, aliquots of a suspension of human erythrocytes in phosphate-buffered saline solution were mixed with aliquots of the test samples and the comparator in microtitre plates and further diluted stepwise to obtain a dilution series down to a dilution factor of 256. As a positive control, an analogue dilution series of the erythrocyte suspension with buffer was prepared wherein the erythrocytes where subsequently lysed completely using a surfactant solution. For each dilution, the degree of haemolysis was quantified by measuring the optical density of haemoglobin by spectrophotometry.

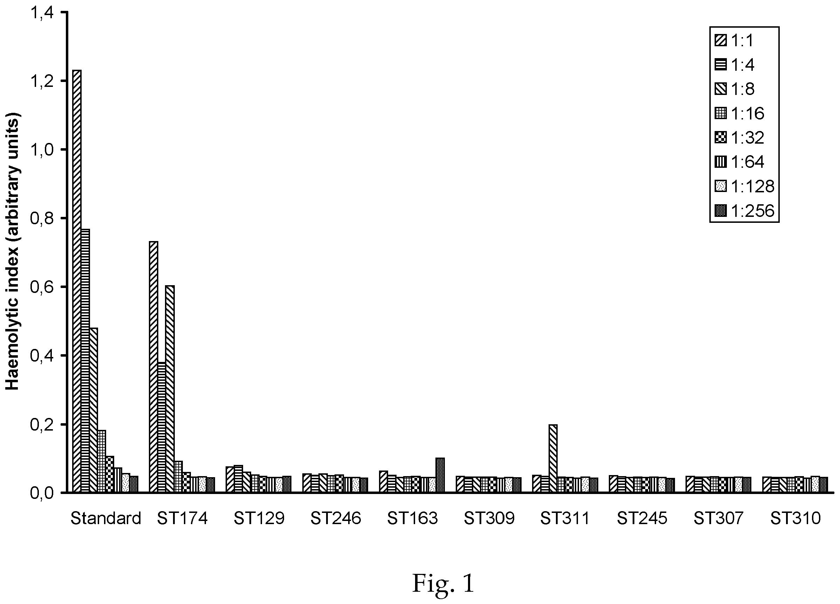

In result, it was found that, in the absence of serum, only the emulsion prepared from propofol without semifluorinated alkanes (ST174) caused significant, concentration-dependent haemolysis. All other emulsion compositions showed no or only negligible haemolysis, regardless of the concentration (see figure 1).

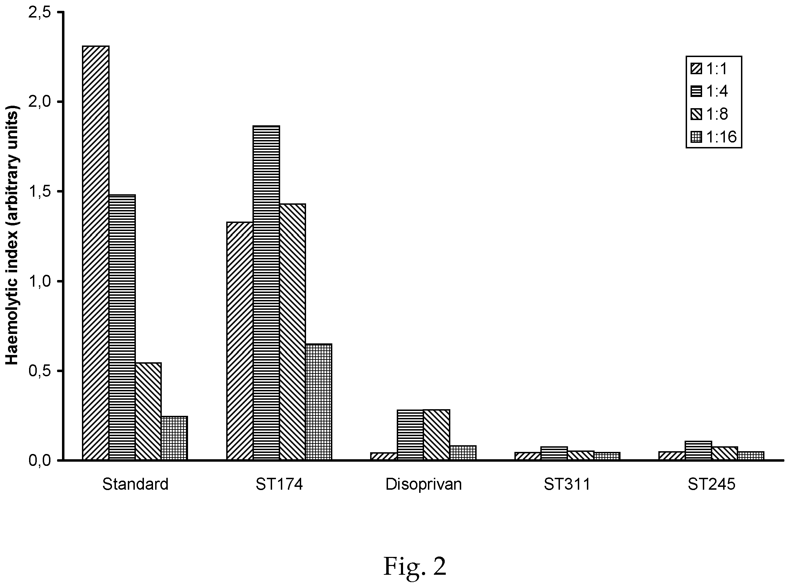

In the presence of serum, the propofol emulsion without semifluorinated alkanes (ST174) again caused significant, concentration-dependent haemolysis, whereas both tested emulsions comprising semifluorinated alkanes (ST245, ST311) showed no haemolysis. Disoprivan® was also tested this time and showed some moderate degree of haemolysis (see figure 2).

**Table 8**

| **Batch code** | **Propofol** | **F6H8** | **S75¹** | **Average droplet size** | |
|---|---|---|---|---|---|
| | | | | **t=0** | **t=84 d** |
| ST174 | 1 wt.-% | - | 8 wt.-% | 138 nm | 141 nm |
| ST129 | 1 wt.-% | 1 wt.-% | 8 wt.-% | 135 nm | 209 nm |
| ST246 | 1 wt.-% | 2 wt.-% | 8 wt.-% | 165 nm | 232 nm |
| ST163 | 1 wt.-% | 3 wt.-% | 8 wt.-% | 188 nm | 247 nm |
| ST309 | 1 wt.-% | 4 wt.-% | 8 wt.-% | 164 nm | 197 nm |
| ST311 | - | 5 wt.-% | 8 wt.-% | 182 nm | 253 nm |
| ST245 | 1 wt.-% | 5 wt.-% | 8 wt.-% | 167 nm | 170 nm |
| ST307 | 1 wt.-% | 6 wt.-% | 8 wt.-% | 167 nm | 182 nm |
| ST310 | 1 wt.-% | 9 wt.-% | 8 wt.-% | 182 nm | 187 nm |

| | | | | | |
|---|---|---|---|---|---|
| ¹ Percentage of S75 relates to the weight of the dispersed phase; all other percentages relate to the total emulsion | | | | | |

These results demonstrate that propofol emulsions based on semifluorinated alkanes as provided according to the invention show excellent blood compatibility which should correspond to a superior tolerability profile. This is in spite of the fact that propofol itself shows poor compatibility with erythrocytes and leads to significant haemolysis. Apparently, the content of semifluorinated alkanes in the compositions of the invention provides protection against haemolysis.

### Example 15

The sedation effectiveness of a propofol emulsion with F6H8 having a strength of 1 wt.-% (batch code ST245, see table 8) was compared to that of Disoprivan® in a rat sedation model. In short, the following parameters were observed and recorded along with the time of occurrence after i.v. injection of a dose corresponding to 10 mg propofol per kg body weight of each formulation: Loss of righting reflex (ta), loss of reaction to toe pinch (i.e. pain) stimulus (tb), start of excitation phase (tex), regain of righting reflex (trr).

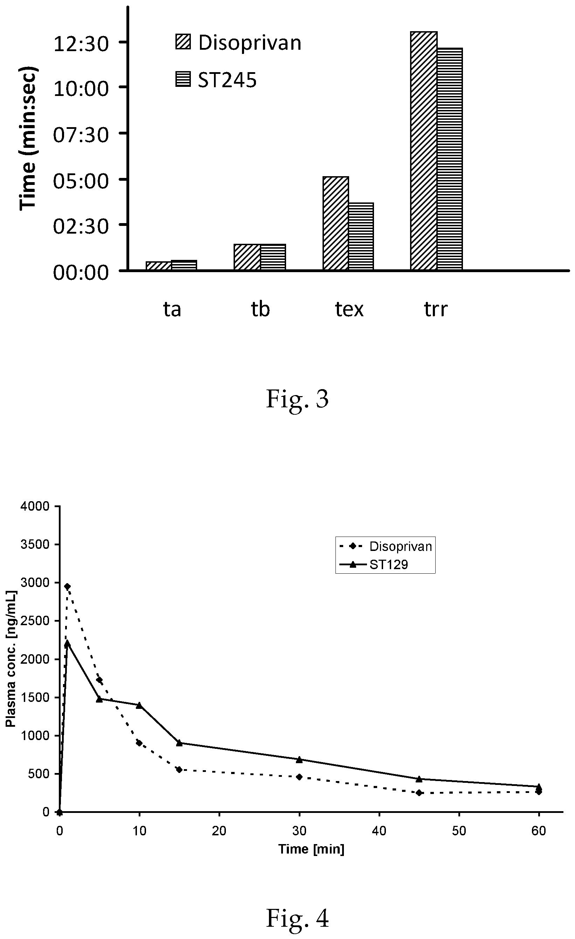

In result, the same effects occurred at similar times after injection, and some minor differences between Disoprivan® and the test emulsion with respect to the start of the excitation phase and the regain of the righting reflex were not statistically significant (see figure 3).

### Example 16

The pharmacokinetics after intravenous injection of a propofol emulsion comprising F6H8 having a propofol strength of 1 wt.-% (batch code ST129, see table 8) in Wistar rats was compared to that of Disoprivan®. As shown in figure 4, the plasma concentration profiles were rather similar for both formulations, with some minor differences with respect to the maximum plasma concentrations, indicating that the test emulsion could be used in a similar manner as Disoprivan®, without requiring dose adaptations.

## Claims

1. A liquid composition in the form of a physically stable O/W-emulsion comprising:
(a) a dispersed phase comprising a semifluorinated alkane according to formula
RFRH;
(b) an aqueous continuous phase, and
(c) at least one surfactant;
wherein:
RF is a linear perfluorinated hydrocarbon segment with 4 to 12 carbon atoms,
RH is a linear alkyl group with 4 to 8 carbon atoms, wherein perfluorinated compounds are absent from the dispersed phase, and wherein the average droplet size of the dispersed phase is below about 1 µm.

2. The composition of claim 1, wherein the semifluorinated alkane is selected from F4H5, F4H6, F4H8, F6H6 and F6H8.

3. The composition of any preceding claim, further **characterised in that** it is sterile and/or heat sterilisable.

4. The composition of any preceding claim, comprising a nonionic surfactant, further **characterised in that** the aqueous continuous phase comprises a salt or ionic compound.

5. The composition of claims 1 to 3, comprising an ionic surfactant, further **characterised in that** the aqueous continuous phase comprises a physiologically acceptable, nonionic osmotic agent.

6. The composition of any preceding claim, wherein the continuous aqueous phase comprises a compound selected from buffers and amino acids.

7. The composition of any preceding claim, wherein the dispersed phase comprises an active pharmaceutical ingredient.

8. The composition of claim 7, wherein the active pharmaceutical ingredient is propofol.

9. The composition of claim 8, wherein the concentration of propofol in the dispersed phase is at least about 10 wt.-%.

10. The composition of claim 8 or 9 for use in the induction and/or maintenance of anaesthesia, or for sedation, wherein the use comprises the parenteral administration of the composition to a patient in need thereof.

11. The composition of any preceding claim, wherein the dispersed phase represents at least about 50 wt.-% of the emulsion.

12. The composition of claims 1 to 11 for use as a medicine.

13. The composition for use as a medicine according to claim 12, wherein the composition is adapted for dermal, mucosal, buccal or sublingual administration.

14. The use of a composition of claims 1 to 8 as a medium for the preservation and/or storage and/or transport of an organ transplant.

## Patentansprüche

1. Eine flüssige Zusammensetzung in der Form einer physikalisch stabilen O/W-Emulsion umfassend:
(a) eine dispergierte Phase umfassend ein semifluoriertes Alkan gemäß Formel
RFRH
(b) eine wässrige kontinuierliche Phase, und
(c) mindestens ein Tensid;
wobei:
RF ein lineares perfluoriertes Kohlenwasserstoff-Segment mit 4 bis 12 Kohlenstoffatomen ist,
RH eine lineare Alkylgruppe mit 4 bis 8 Kohlenstoffatomen ist, wobei die dispergierte Phase keine perfluorierte Verbindungen enhält, und wobei die durchschnittliche Tröpfchengröße der dispergierten Phase unter etwa 1 µm ist.

2. Die Zusammensetzung nach Anspruch 1, wobei das semifluorierte Alkan ausgewählt ist aus F4H5, F4H6, F4H8, F6H6 und F6H8.

3. Die Zusammensetzung nach einem der vorhergehenden Ansprüche, weiterhin dadurch charakterisiert, dass sie steril und/oder hitzesterilisierbar ist.

4. Die Zusammensetzung nach einem der vorhergehenden Ansprüche, umfassend ein nichtionisches Tensid, weiterhin dadurch charakterisiert, dass die wässrige kontinuierliche Phase ein Salz oder eine ionische Verbindung umfasst.

5. Die Zusammensetzung nach den Ansprüchen 1 bis 3, umfassend ein ionisches Tensid, weiterhin dadurch charakterisiert, dass die wässrige kontinuierliche Phase einen physiologisch akzeptablen, nichtionischen osmotischen Wirkstoff umfasst.

6. Die Zusammensetzung nach einem der vorhergehenden Ansprüche, wobei die kontinuierliche wässrige Phase eine Verbindung ausgewählt aus Puffern und Aminosäuren umfasst.

7. Die Zusammensetzung nach einem der vorhergehenden Ansprüche, wobei die dispergierte Phase einen pharmazeutischen Wirkstoff umfasst.

8. Die Zusammensetzung nach Anspruch 7, wobei der pharmazeutische Wirkstoff Propofol ist.

9. Die Zusammensetzung nach Anspruch 8, wobei die Konzentration des Propofols in der dispergierten Phase mindestens etwa 10 Gew.-% ist.

10. Die Zusammensetzung nach Anspruch 8 oder 9 zur Verwendung bei der Einleitung und/oder des Erhalts von Anästhesien oder zu Sedierungen, wobei die Verwendung die parenterale Verabreichung der Zusammensetzung an einen behandlungsbedürftigen Patienten umfasst.

11. Die Zusammensetzung nach einem der vorhergehenden Ansprüche, wobei die dispergierte Phase mindestens etwa 50 Gew.-% der Emulsion ausmacht.

12. Die Zusammensetzung nach Anspruch 1 bis 11 zur Verwendung als Arzneimittel.

13. Die Zusammensetzung zur Verwendung als Arzneimittel nach Anspruch 12, wobei die Zusammensetzung für dermale, mukosale, bukkale oder sublinguale Verabreichung angepasst ist.

14. Die Verwendung der Zusammensetzung nach Anspruch 1 bis 8 als Mittel zur Konservierung und/oder Aufbewahrung und/oder zum Transport eines Organtransplantats.

## Revendications

1. Composition liquide se présentant sous la forme d'une émulsion H/E physiquement stable comprenant :
(a) une phase dispersée comportant un alcane semi-fluoré ayant la formule
RFRH
(b) une phase continue aqueuse, et
(c) au moins un tensioactif;
RF étant un segment hydrocarboné perfluoré linéaire ayant 4 à 12 atomes de carbone,
RH étant un groupe alkyle linéaire ayant 4 à 8 atomes de carbone, dans laquelle les composés perfluorés sont absents de la phase dispersée, et la taille moyenne des gouttelettes de la phase dispersée étant inférieure à environ 1 µm.

2. Composition selon la revendication 1, dans laquelle l'alcane semi-fluoré est choisi parmi F4H5, F4H6, F4H8, F6H6 et F6H8.

3. Composition selon l'une quelconque des revendications précédentes, **caractérisée en outre en ce qu'**elle est stérile et/ou peut être stérilisée à la chaleur.

4. Composition selon l'une quelconque des revendications précédentes, comprenant un tensioactif non ionique, **caractérisée en outre en ce que** la phase continue aqueuse comprend un sel ou un composé ionique.

5. Composition selon les revendications 1 à 3, comprenant un tensioactif ionique, **caractérisée en outre en ce que** la phase continue aqueuse comprend un agent osmotique non ionique physiologiquement acceptable.

6. Composition selon l'une quelconque des revendications précédentes, dans laquelle la phase continue aqueuse comprend un composé choisi parmi les tampons et les acides aminés.

7. Composition selon l'une quelconque des revendications précédentes, dans laquelle la phase dispersée comprend un principe actif pharmaceutique.

8. Composition selon la revendication 7, dans laquelle le principe actif pharmaceutique est le propofol.

9. Composition selon la revendication 8, dans laquelle la concentration de propofol dans la phase dispersée est d'au moins 10 % en poids environ.

10. Composition selon la revendication 8 ou 9 destinée à être utilisée pour induire et/ou maintenir l'anesthésie, ou pour la sédation, l'utilisation comprenant l'administration par voie parentérale de la composition chez un patient en ayant besoin.

11. Composition selon l'une quelconque des revendications précédentes, dans laquelle la phase dispersée représente au moins 50% en poids environ de l'émulsion.

12. Composition selon les revendications 1 à 11 destinée à être utilisée comme médicament.

13. Composition destinée à être utilisée comme médicament selon la revendication 12, dans laquelle la composition est adaptée à l'administration cutanée, muqueuse, buccale ou sublinguale.

14. Utilisation d'une composition selon les revendications 1 à 8 comme milieu pour la conservation et/ou le stockage et/ou le transport d'une greffe d'organe.
